# FASCICULE DE BREVET EUROPEEN

(11) **EP 4 586 956 B1**
(45) Date de publication et mention de la délivrance du brevet: **01.07.2026**
(21) Numéro de dépôt: 23768853.6
(22) Date de dépôt: 11.09.2023
(51) Int. Cl.: A61C 8/00, A61B 17/86, A61B 50/30

(54) **DISPOSITIF DE CONDITIONNEMENT POUR UNE PROTHÈSE DENTAIRE**
VERPACKUNGSVORRICHTUNG FÜR EINE ZAHNPROTHESE
PACKAGING DEVICE FOR A DENTAL PROSTHESIS

(30) Priorité: 12.09.2022 FR 2209112
(43) Date de publication de la demande: 23.07.2025
(73) Titulaire: Euroteknika, 74700 Sallanches (FR)
(72) Inventeur: LO SAVIO VOGEL, Rudy, 74700 DOMANCY (FR); LANCIEUX, Cédric, 74120 MEGÈVE (FR)
(74) Mandataire: Novaimo
(86) Numéro de dépôt international: PCT/EP2023/074921
(87) Numéro de publication internationale: WO 2024/056614

(56) Documents cités:
- EP-A1- 3 995 104
- WO-A1-2015/179931
- US-A1- 2007 295 620
- US-A1- 2021 212 788

## Description

### Domaine technique de l'invention

L'invention concerne le domaine des dispositifs de conditionnement pour une prothèse dentaire, notamment pour un implant dentaire. L'invention concerne également un procédé de fabrication d'un tel dispositif de conditionnement, un procédé de conditionnement d'une prothèse dentaire dans un tel dispositif de conditionnement, et un procédé de déconditionnement d'une prothèse dentaire conditionnée dans un tel dispositif de conditionnement.

### Etat de la technique antérieure

Les prothèses dentaires, telles que les implants dentaires, sont destinées à être intégrées dans la bouche d'un patient afin de restaurer sa dentition. Les prothèses dentaires doivent présenter une qualité et une propreté irréprochable au moment de leur intégration en bouche. Entre leur fabrication et leur utilisation par un dentiste, les prothèses dentaires sont généralement conditionnées dans des dispositifs de conditionnement individuels. Les dispositifs de conditionnement doivent permettre de protéger les prothèses dentaires de tout risque d'endommagement ou de contamination entre leur fabrication et leur utilisation. Les publications US2021/212788A1, US2007295620A1 et EP0963738A1 divulguent des exemples de dispositifs de conditionnement. Les dispositifs de conditionnement connus sont généralement trop complexes et/ou peu pratiques à utiliser, en particulier dans le cadre de soins dentaires où le dentiste doit pouvoir déconditionner la prothèse dentaire de manière efficace et rapide. Aucun des documents cités ne décrit un dispositif de conditionnement avec au moins un bras relié à une embase et le mécanisme d'ouverture selon l'invention.

### Présentation de l'invention

Le but de l'invention est de fournir un dispositif de conditionnement pour une prothèse dentaire remédiant aux inconvénients ci-dessus et améliorant les dispositifs de conditionnement connus de l'art antérieur.

Plus précisément, un premier objet de l'invention est un dispositif de conditionnement facile à fabriquer et à utiliser.

### Résumé de l'invention

L'invention se rapporte à un dispositif de conditionnement pour une prothèse dentaire,
comprenant:
- une embase comprenant un support destiné à maintenir la prothèse dentaire,
- une enveloppe formée d'une première partie et d'une deuxième partie, et
- au moins un bras comprenant une première extrémité et une deuxième extrémité, la première extrémité étant reliée à l'embase, la deuxième extrémité étant reliée à la première partie de l'enveloppe,
l'embase comprend une forme globalement allongée, la première extrémité de l'au moins un bras étant reliée à une première extrémité de l'embase, le support étant agencé à une deuxième extrémité de l'embase, la deuxième extrémité de l'embase étant opposée à la première extrémité de l'embase, le dispositif de conditionnement étant apte à passer d'une configuration fermée à une configuration ouverte, la première partie et la deuxième partie de l'enveloppe étant rapprochée l'une de l'autre de manière à envelopper la prothèse dentaire lorsque le dispositif de conditionnement est en configuration fermée, la première partie et la deuxième partie de l'enveloppe étant écartées l'une de l'autre de manière à accéder à la prothèse dentaire lorsque le dispositif de conditionnement est en configuration ouverte, l'embase comprenant au moins un moyen de pivot pour l'au moins un bras, un effort appliqué sur l'au moins un bras entre sa première extrémité et l'au moins un moyen de pivot faisant fléchir l'au moins un bras en direction de l'embase, la flexion de l'au moins un bras entraînant le passage du dispositif de conditionnement de sa configuration fermée à sa configuration ouverte.

Le dispositif de conditionnement peut comprendre un premier bras et un deuxième bras, chaque bras comprenant une première extrémité et une deuxième extrémité, la première extrémité de chaque bras étant reliée à l'embase, la deuxième extrémité du premier bras étant reliée à la première partie de l'enveloppe, la deuxième extrémité du deuxième bras étant reliée à la deuxième partie de l'enveloppe, l'embase comprenant un premier moyen de pivot pour le premier bras et un deuxième moyen de pivot pour le deuxième bras, un effort appliqué sur le premier bras entre sa première extrémité et le premier moyen de pivot et un effort appliqué sur le deuxième bras entre sa première extrémité et le deuxième moyen de pivot entraînant le passage du dispositif de conditionnement de sa configuration fermée à sa configuration ouverte.

Le dispositif de conditionnement peut être une pièce monobloc obtenue par injection plastique.

L'embase peut comprendre au moins un moyen de retenue coopérant avec l'au moins un bras pour maintenir le dispositif de conditionnement en configuration fermée.

Le moyen de retenue peut comprendre au moins une patte déformable, la patte étant destinée à se déformer pour permettre le déplacement de l'au moins un bras lorsque le dispositif de conditionnement passe d'une configuration originelle à l'issue de sa fabrication à sa configuration fermée.

Le dispositif de conditionnement peut comprendre une mémoire de forme tendant à le faire passer vers sa configuration fermée.

L'embase peut comprendre en outre au moins un logement pour une vis de fixation apte à coopérer avec la prothèse dentaire. La première partie et la deuxième partie de l'enveloppe peuvent être deux demi-coques de l'enveloppe. La première partie et/ou la deuxième partie de l'enveloppe peuvent être fabriquées en matériau plastique transparent et peuvent comporter des graduations en relief configurées pour estimer une taille de la prothèse dentaire.

Le dispositif de conditionnement peut comprendre un clip de fixation agencé entre les deux parties de l'enveloppe pour maintenir le dispositif de conditionnement en configuration fermée.

L'invention se rapporte également à un kit comprenant un dispositif de conditionnement tel que défini précédemment et une prothèse dentaire fixée au support de l'embase.

L'invention se rapporte également à un procédé de fabrication d'un dispositif de conditionnement tel que défini précédemment, le dispositif de conditionnement étant fabriqué par injection plastique dans une configuration originelle dans laquelle l'au moins un bras présente une forme en arc de cercle et dans laquelle l'au moins un bras est écarté de l'au moins un moyen de pivot.

L'invention se rapporte également à un procédé de conditionnement d'une prothèse dentaire dans un dispositif de conditionnement tel que défini précédemment, le procédé de conditionnement comprenant :
- la fabrication par injection plastique du dispositif de conditionnement, puis
- la fixation d'une prothèse dentaire au support de l'embase, puis
- le rapprochement de la première partie de l'enveloppe et de la deuxième partie de l'enveloppe autour de la prothèse dentaire.

L'invention se rapporte également à un procédé de déconditionnement d'une prothèse dentaire conditionnée dans un dispositif de conditionnement tel que défini précédemment, comprenant une pression contre l'au moins un bras entre sa première extrémité et l'au moins un moyen de pivot pour faire fléchir l'au moins un bras en direction de l'embase, la flexion de l'au moins un bras entraînant l'ouverture du dispositif de conditionnement.

### Présentation des figures

Ces objets, caractéristiques et avantages de la présente invention seront exposés en détail dans la description suivante d'un mode de réalisation particulier fait à titre non-limitatif en relation avec les figures jointes parmi lesquelles :
La figure 1 est une vue en perspective d'un dispositif de conditionnement selon un mode de réalisation de l'invention, le dispositif de conditionnement étant dans une configuration originelle.
La figure 2 est une vue en perspective du dispositif du conditionnement dans une configuration fermée.
La figure 3 est une vue de profil du dispositif du conditionnement en configuration originelle.
La figure 4 est une vue de profil du dispositif du conditionnement en configuration fermée.
La figure 5 est une vue schématique de profil de la cinématique de fermeture du dispositif du conditionnement autour d'une prothèse dentaire.
La figure 6 est une vue de profil et en transparence du dispositif du conditionnement en configuration fermée, la prothèse dentaire étant maintenu dans un support du dispositif de conditionnement.
La figure 7 est une vue schématique de profil du dispositif de conditionnement au cours de son ouverture.
La figure 8 est une vue schématique de profil du dispositif de conditionnement en configuration ouverte.
La figure 9 en une vue en perspective et en éclaté d'un kit comprenant le dispositif de conditionnement, la prothèse dentaire et deux vis de fixation aptes à coopérer avec la prothèse dentaire.
La figure 10 est une vue de profil d'une première variante de réalisation d'un dispositif de conditionnement.
La figure 11 est une vue de profil d'une deuxième variante de réalisation d'un dispositif de conditionnement, le dispositif de conditionnement étant en configuration originelle.
La figure 12 est une vue de profil de la deuxième variante de réalisation du dispositif de conditionnement, le dispositif de conditionnement étant en configuration fermée.

### Description détaillée

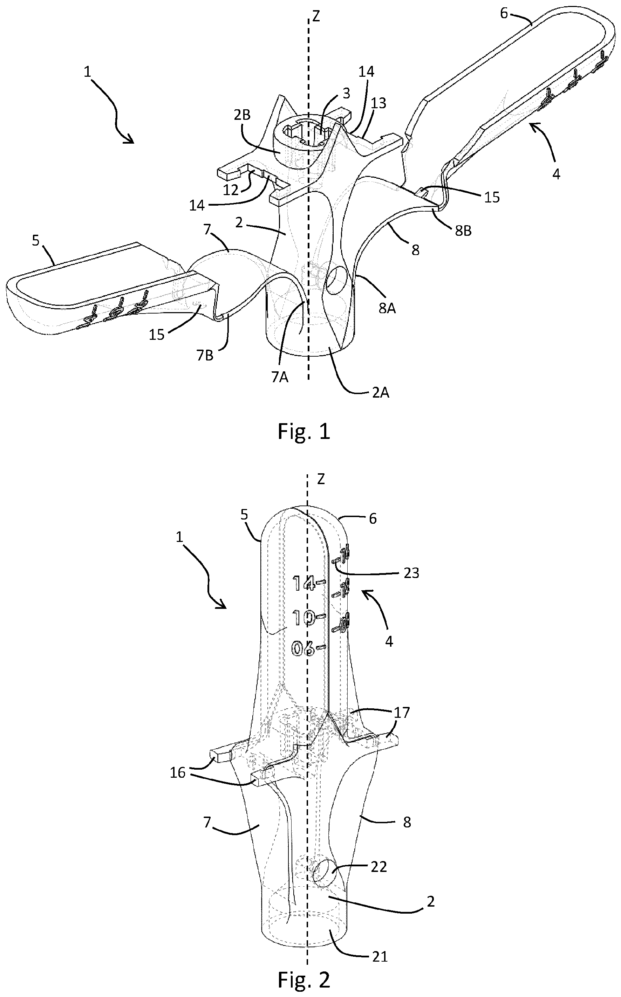

La figure 1 illustre un dispositif de conditionnement 1 pour une prothèse dentaire selon un mode de réalisation de l'invention. Le dispositif de conditionnement 1 est destiné protéger une prothèse dentaire entre sa fabrication et son utilisation par un dentiste. Le dispositif de conditionnement 1 est un dispositif de conditionnement unitaire, c'est-à-dire qu'il est destiné à envelopper une unique prothèse dentaire. En variante, le dispositif de conditionnement pourrait contenir plusieurs prothèses dentaires. En particulier, la prothèse dentaire est un implant dentaire. L'implant dentaire est destiné à être vissé dans une structure osseuse du patient. En raison de son intégration dans l'os du patient, l'implant dentaire requiert un dispositif de conditionnement permettant une protection optimale contre tout risque de contamination. Ainsi, le dispositif de conditionnement est destiné à envelopper de manière hermétique ou quasi-hermétique la prothèse dentaire, notamment de sorte à empêcher un contact direct entre la prothèse dentaire et les doigts d'un utilisateur. En variante la prothèse dentaire pourrait être différente d'un implant dentaire, par exemple un pilier dentaire, une couronne dentaire ou un bridge dentaire.

Le dispositif de conditionnement 1 comprend une embase 2 comprenant un support 3 destiné à maintenir la prothèse dentaire, une enveloppe 4 formée d'une première partie 5 et d'une deuxième partie 6, et deux bras 7, 8. Un premier bras 7 s'étend entre l'embase 2 et la première partie 5 de l'enveloppe 4. De même, un deuxième bras 8 s'étend entre l'embase 2 et la deuxième partie 6 de l'enveloppe 4. Le premier bras 7 comprend donc une première extrémité 7A reliée à l'embase 2 et une deuxième extrémité 7B, opposée à la première extrémité 7A, reliée à la première partie 5 de l'enveloppe 4. De même, le deuxième bras 8 comprend une première extrémité 8A reliée à l'embase 2 et une deuxième extrémité 8B, opposée à la première extrémité 8A, reliée à la deuxième partie 6 de l'enveloppe 4. Les deux parties 5 et 6 de l'enveloppe sont donc reliées à l'embase 2 respectivement par l'intermédiaire des deux bras 7 et 8.

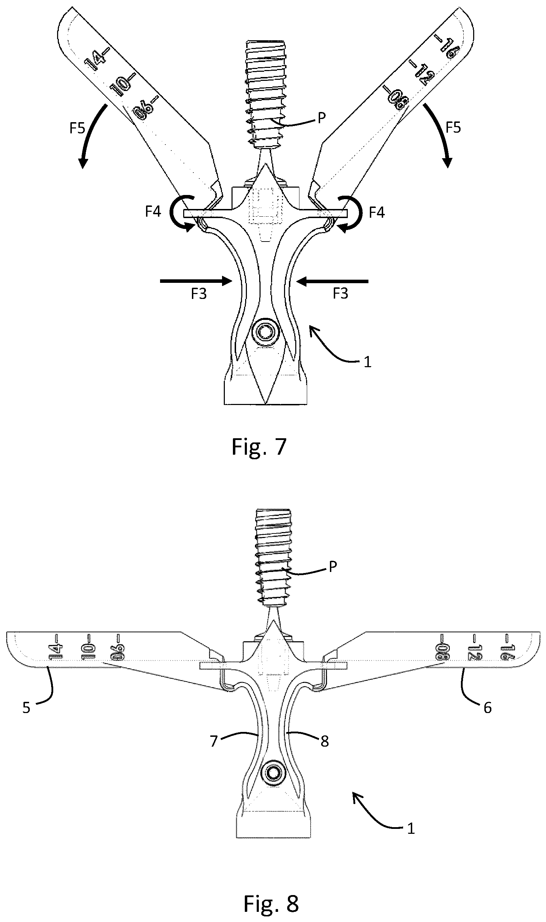

Sur la figure 1, le dispositif de conditionnement est représenté en configuration originelle, c'est-à-dire dans un état tel que le dispositif de conditionnement est fabriqué, sans déformation. Le dispositif de conditionnement est apte à passer de sa configuration originelle à une configuration fermée (illustrée sur la figure 2). En configuration fermée, la première partie 5 et la deuxième partie 6 de l'enveloppe 4 sont rapprochées l'une de l'autre de manière à envelopper la prothèse dentaire. Notamment, les deux parties 5 et 6 sont en contact l'une contre l'autre le long d'une ligne de contact. En configuration ouverte (illustrée sur la figure 8), la première partie 5 et la deuxième partie 6 de l'enveloppe 4 sont écartées l'une de l'autre, sans contact entre elles de manière à accéder à la prothèse dentaire fixée au support 3. A minima, le dispositif de conditionnement 1 est apte à passer une fois de sa configuration originelle à sa configuration fermée (lors du conditionnement de la prothèse dentaire) puis une fois de sa configuration fermée à sa configuration ouverte (lors du déconditionnement de la prothèse dentaire). Le dispositif de conditionnement 1
peut ainsi être à usage unique. En variante, le dispositif de conditionnement 1 pourrait aussi être réutilisé plusieurs fois.

Selon le mode de réalisation présenté, l'enveloppe 4 présente une forme globalement cylindrique avec une base circulaire lorsque le dispositif de conditionnement est en configuration fermée. La première partie 5 et la deuxième partie 6 de l'enveloppe sont deux demi-coques de l'enveloppe de forme sensiblement identique. Notamment, elles comprennent chacune la forme d'un demi-cylindre. En variante, toute autre forme pourrait être proposée comme par exemple une forme cylindrique avec une base polygonale, notamment rectangulaire, ou encore une forme sphérique ou ovoïde. On peut définir un axe Z comme étant un axe parallèle à une génératrice de la forme cylindrique de l'enveloppe et passant par le centre de celle-ci. L'embase 2 comprend une forme globalement allongée suivant l'axe Z. la première extrémité 7A, 8A de chaque bras 7, 8 est reliée à une première extrémité 2A de l'embase 2. Le support 3 est agencé à une deuxième extrémité 2B de l'embase, la deuxième extrémité 2B étant opposée à la première extrémité 2A. En particulier, le support 3 se présente sous la forme d'une ouverture borgne s'étendant suivant l'axe Z. Cette ouverture borgne peut avoir une section profilée permettant l'insertion et l'extraction de la prothèse dentaire par un simple mouvement de translation parallèlement à l'axe Z. Dans le cadre de la description on suppose que l'axe Z est un axe vertical et que l'embase repose sur un plan horizontal. Les termes "inférieur" et "supérieur" se rapportent à un agencement selon l'axe Z. Le dispositif de conditionnement est avantageusement symétrique ou quasi-symétrique par rapport à un plan passant par l'axe Z.

L'embase 2 peut également s'inscrire dans une forme cylindrique centrée sur l'axe Z. Avantageusement l'embase 2 comprend une forme échancrée permettant à chaque bras 7, 8 de fléchir en direction de l'embase 2. L'embase 2 comprend ainsi une partie centrale 9 relativement mince encadrée par une partie inférieure 10 et une partie supérieure 11 plus larges. L'embase 2 comprend ainsi globalement la forme d'un sablier.

L'embase 2 comprend deux moyens de pivot 12 et 13 respectivement pour chacun des deux bras 7 et 8. Les deux moyens de pivot 12, 13 sont agencés au niveau de la deuxième extrémité 2B de l'embase, c'est-à-dire au niveau de sa partie supérieure 11. Les deux moyens de pivot 12, 13 peuvent se présenter sous la forme d'une plaque s'étendant sensiblement perpendiculairement à l'axe Z. Lorsque le dispositif de conditionnement est en configuration fermée, les deuxièmes extrémités 7B et 8B de chaque bras peuvent se trouver en contact ou à faible distance respectivement des moyens de pivot 12 et 13. Les moyens de pivots 12, 13 sont destinés à former des points de pivot pour chacun des deux bras 7 et 8. Un fléchissement du premier bras 7 entre sa première extrémité 7A et le premier moyen de pivot 12 entraîne le pivotement de la première partie 5 de l'enveloppe 7 autour du premier moyen de pivot 12. De même, un fléchissement du deuxième bras 8 entre sa première extrémité 8A et le deuxième moyen de pivot 13 entraîne le pivotement de la deuxième partie 6 de l'enveloppe 7 autour du deuxième moyen de pivot 13.

Le dispositif de conditionnement comprend également un moyen de retenue configuré pour maintenir le dispositif de conditionnement en configuration fermée. Ce moyen de retenue comprend deux pattes 16 pour retenir la première partie 5 de l'enveloppe 4 en configuration fermée, et deux pattes 17 pour retenir la deuxième partie 6 de l'enveloppe 4 en configuration fermée. Les pattes 16 et 17 sont destinées à coopérer respectivement avec les deuxièmes extrémités 7B et 8B de chaque bras. Les pattes 16, 17 forment des clips de fixation apte à exercer un effort de retenue au dos de chaque bras. Les pattes 16 et 17 permettent ainsi de maintenir respectivement les bras 7 et 8 en appui contre les moyens de pivot 12 et 13 ou à faible distance des moyens de pivot 12 et 13. En variante, une seule patte 16, 17 pourrait être suffisante pour retenir la première partie 5 ou la deuxième partie 6 en configuration fermée. Chaque patte 16, 17 est destinée à se déformer pour permettre le déplacement de l'au moins un bras lorsque le dispositif de conditionnement passe de sa configuration originelle à sa configuration fermée.

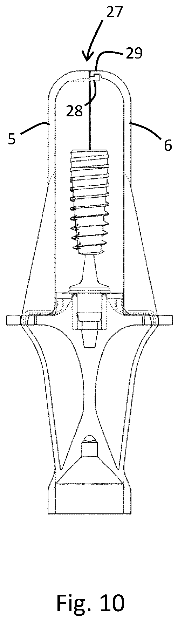

Selon une variante de réalisation illustrée sur la figure 10, le moyen de retenue décrit plus haut peut être remplacé ou complété par un deuxième moyen de retenue. Le deuxième moyen de retenue est formé par un clip de fixation 27 agencé entre les deux parties 5 et 6 de l'enveloppe 4. Les parties 5, 6 de l'enveloppe 4 comprennent chacune une surface d'emboitement 28, 29 coopérant ensemble pour maintenir le dispositif de conditionnement en configuration fermée. Ces surfaces d'emboitement 28, 29 peuvent s'étendre le long de toute la ligne de contact entre la première partie 5 et la deuxième partie 6 de l'enveloppe. Dans ce cas, elles peuvent avantageusement former une chicane qui améliore l'étanchéité du dispositif de conditionnement. Alternativement, les surfaces d'emboitement peuvent s'étendre le long d'une partie seulement de cette ligne de contact, par exemple uniquement au niveau de l'extrémité supérieure des parties 5 et 6 de l'enveloppe 4. Ainsi le clip de fixation est simple à réaliser et l'effort nécessaire pour ouvrir le dispositif de conditionnement est réduit. Selon une autre variante, les deux parties 5 et 6 pourraient également être faiblement soudées ou collées l'une à l'autre lorsque le dispositif de conditionnement est en configuration fermée

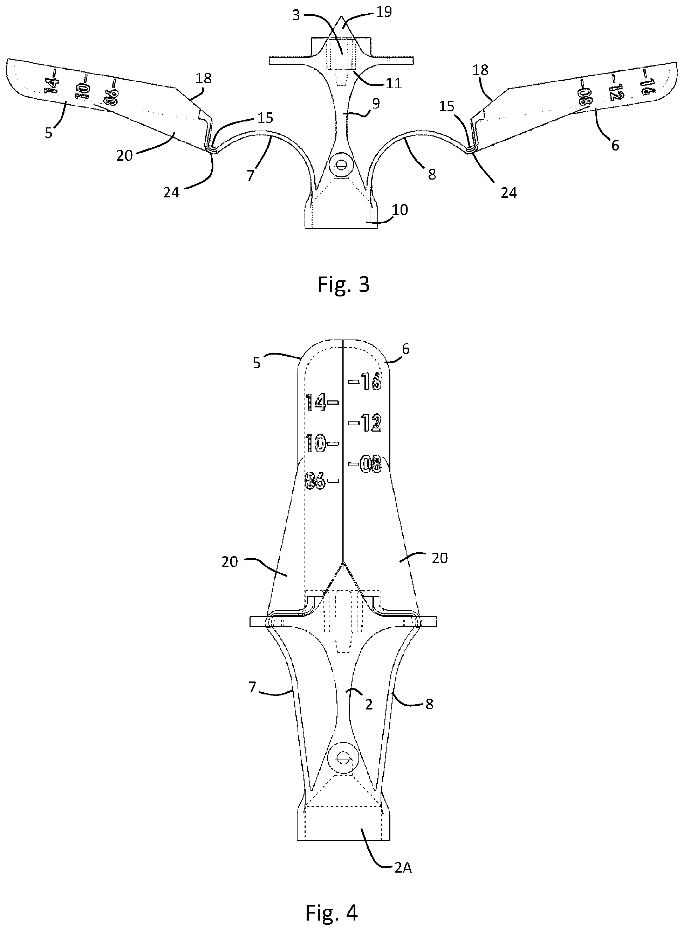

Comme illustré sur la figure 3, chacune des deux parties 5 et 6 de l'enveloppe présentent des évidements 18, ou découpes 18, au niveau de leur extrémité inférieure. Ces évidements 18 permettent à chacune des deux parties 5 et 6 de pivoter entre la configuration originelle ou la configuration ouverte et la configuration fermée sans interférer avec les moyens de pivot 12 et 13. La partie supérieure 11 de l'embase 2 comprend deux flancs 19 encadrant le support 3 et surmontant les plaques dans lesquelles sont formés les moyens de pivot 12 et 13. Les deux flancs 19 présentent une forme adaptée à combler les évidements 18 lorsque le dispositif de conditionnement est en configuration fermée. En l'espèce, les deux flancs 19 présentent une forme globalement triangulaire.

Lorsque le dispositif de conditionnement est en configuration originelle, les deux bras 7 et 8 présentent une forme en arc de cercle. Les deux parties 5 et 6 de l'enveloppe s'étendent sensiblement horizontalement et en dessous du niveau de la prothèse dentaire fixée sur le support 3. La prothèse dentaire est alors très largement accessible. Lorsque le dispositif de conditionnement est en configuration fermée, les deux bras 7 et 8 comprennent une portion de forme rectiligne. Cette forme rectiligne s'étend depuis la première extrémité 2A de l'embase jusqu'aux moyens de pivot 12, 13. Chaque bras 7, 8 comprend aussi une courbure 24 respectivement autour du moyen de pivot 12, 13 au niveau de sa deuxième extrémité 7B, 8B. Les bras 7, 8 se terminent ainsi par des portions horizontales qui s'étendent depuis les moyens de pivot 12, 13 jusqu'à l'extrémité inférieure de chaque partie 5, 6 de l'enveloppe.

Avantageusement, le dispositif de conditionnement comprend des premières nervures 20 s'étendant sur une face extérieure de chaque partie 5, 6 de l'enveloppe jusqu'à son bras 7, 8 respectif. Le dispositif de conditionnement comprend également des deuxièmes nervures 15 agencées à l'intérieur des courbures 24 de chaque bras 7, 8. La plaque dans laquelle sont formés les deux moyens de pivot 12, 13 comprend avantageusement deux encoches 14 dans lesquelles les deuxièmes nervures 15 se positionnent lorsque le dispositif de conditionnement est en configuration fermée. Les premières nervures 20 et les deuxièmes nervures 15 permettent de rigidifier l'interface entre chaque partie 5, 6 de l'enveloppe 4 et son bras 7, 8 respectif, de manière à ce qu'un pivotement de la deuxième extrémité de chaque bras induise bien un pivotement de la partie d'enveloppe correspondante. En variante, le dispositif de conditionnement pourrait ne comprendre que les premières nervures 20 ou que les deuxièmes nervures 15, ou toute autre forme de nervure, ou même aucune nervure, pourvu que la liaison entre chaque bras et la partie d'enveloppe correspondante soit suffisamment rigide, par exemple grâce à des épaisseurs de matière suffisamment importantes.

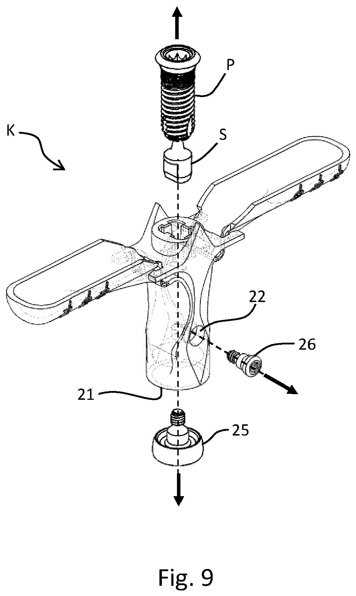

Selon un perfectionnement de l'invention, l'embase 2 comprend au moins un logement pour une vis de fixation apte à coopérer avec la prothèse dentaire. En l'espèce, l'embase 2 comprend deux logements 21, 22 dans lesquels peuvent être placés deux vis de fixation 25, 26 de tailles différentes. Les logements sont des ouvertures borgnes de section circulaire. Un premier logement 21 s'étend dans la partie inférieure 10 de l'embase parallèlement à l'axe Z. Un deuxième logement 22 s'étend également dans la partie inférieure 10 de l'embase, perpendiculairement à l'axe Z, entre les deux bras 7 et 8. On peut ainsi fournir un kit K, illustré sur la figure 9 comprenant la prothèse dentaire P fixée au support 3 et deux vis de fixation 25, 26 agencées dans chacun des deux logements 21, 22. Alternativement, le kit pourrait ne comprendre qu'une seule des deux vis de fixation ou même tout autre accessoire destiné à coopérer avec la prothèse dentaire.

Avantageusement, le dispositif de conditionnement est un élément monobloc fabriqué par injection plastique. Il est donc particulièrement simple à fabriquer et bon marché. Par définition, le dispositif de conditionnement est fabriqué en configuration originelle, c'est-à-dire dans la configuration des figures 1, 3 et 5. D'une part ceci présente l'avantage que le dispositif de conditionnement est immédiatement prêt à recevoir une prothèse dentaire dans son support. D'autre part, ceci confère au dispositif de conditionnement une mémoire de forme correspondant à sa configuration originelle. Les bras 7 et 8 présentent ainsi une élasticité tendant à déplacer chaque partie 5, 6 de l'enveloppe vers une position correspondant à la configuration fermée du dispositif de conditionnement, après que les premières extrémités 7A, 8A de chaque bras soient clipsés dans les clips de fixation formés par les pattes 16 et17. Ceci permet un bon maintien en configuration fermée du dispositif de conditionnement.

La première partie 5 et/ou la deuxième partie 6 de l'enveloppe 4 peuvent être fabriquées en matériau plastique transparent et peuvent comporter des graduations 23 en relief configurées pour estimer une taille de la prothèse dentaire contenue dans l'enveloppe 4. Les graduations 23 peuvent être formées en relief positif (c'est-à-dire en saillie) ou en relief négatif (c'est-à-dire en creux). Un utilisateur peut ainsi comparer la hauteur de la prothèse dentaire maintenue dans le support 3 aux graduations 23 et en estimer la taille sans avoir à ouvrir l'enveloppe 4. Les graduations 23 peuvent être formées directement lors du procédé d'injection plastique du dispositif de conditionnement. Ainsi l'intégration de ces graduations 23 ne requiert pas d'opération de marquage spécifique.

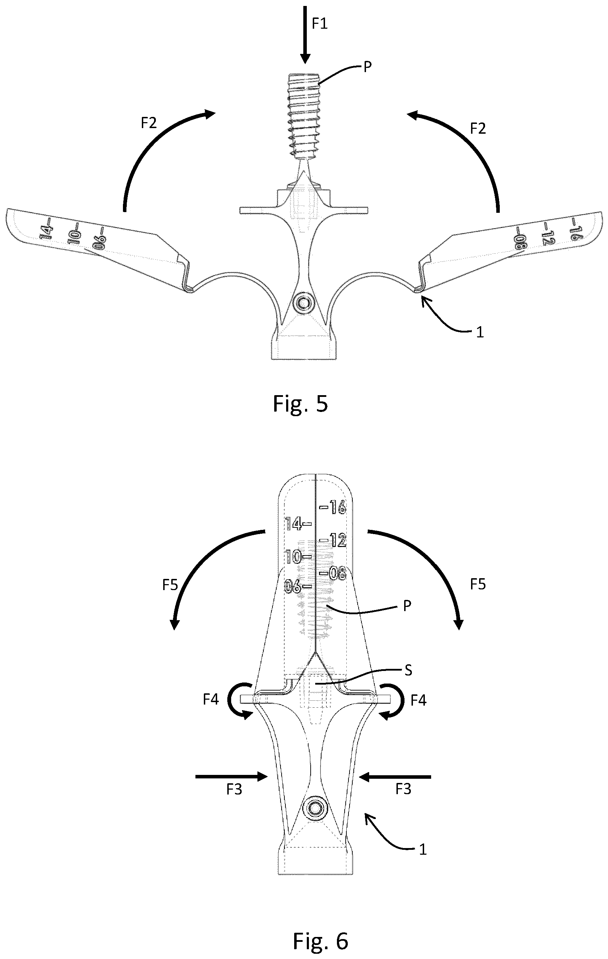

En référence à la figure 5, pour conditionner une prothèse dentaire P dans le dispositif de conditionnement 1, on peut procéder de la manière suivante. On fabrique le dispositif de conditionnement par injection plastique. En parallèle, on fabrique la prothèse dentaire P. Selon le mode de réalisation illustré sur la figure 5, la prothèse dentaire P est un implant dentaire destiné à être vissé dans une structure osseuse du patient. L'implant dentaire est relié à une partie sécable S (bien visible sur la figure 9) par l'intermédiaire de laquelle il est fixé au support 3. En variante, l'implant dentaire pourrait ne pas être relié à une telle partie sécable et être directement fixé au support 3. Toutefois, l'utilisation d'une partie sécable présente l'avantage que la prothèse dentaire P est conservée sans contact avec le dispositif de conditionnement 1, ce qui évite tout risque de contamination. La partie sécable S est insérée dans le support 3 par un simple mouvement de translation parallèlement à l'axe Z, comme indiqué par la flèche F1. Ensuite, on referme la première partie 5 et la deuxième partie 6 de l'enveloppe autour de la prothèse dentaire P autour de la prothèse dentaire. Les deux parties 5 et 6 pivotent d'environ un quart de tour en direction de l'axe Z, comme indiqué par les flèches F2. Lorsque le dispositif de conditionnement est sur le point d'atteindre sa configuration fermée, les pattes 16 et 17 se déforment légèrement pour permettre le passage des bras 7 et 8 contre les moyens de pivot 12 et 13. Le cas échéant, les surfaces d'emboitement 28, 29 s'engagent l'une contre l'autre. Le dispositif de conditionnement est ainsi bien maintenu en configuration fermée. On obtient ainsi un kit K comprenant le dispositif de conditionnement 1 et la prothèse dentaire P fixée au support 3 du dispositif de conditionnement. Le kit K peut être complété par la fixation d'une vis 25 ou 26 ou les deux vis 25 et 26 dans les logements 21 et 22 prévus à cet effet. La prothèse dentaire est ainsi bien protégée et peut être acheminée de son site de fabrication vers son site d'utilisation et conservée sans risque de contamination.

Ensuite, lorsqu'un dentiste souhaite installer une prothèse dentaire en bouche du patient, il peut tout d'abord facilement sélectionner la bonne taille de prothèse dentaire grâce aux graduations 23. Ensuite, le dispositif de conditionnement peut avantageusement être manipulé à une seule main. Le premier bras 7 et le deuxième bras 8 peuvent être pressés ou pincés l'un contre l'autre au moyen du pouce et de l'index d'une seule main, sensiblement entre leurs deux extrémités, comme indiqué par les flèches F3. Ceci provoque la flexion des deux bras 7 et 8 en direction de l'embase. Les deux bras 7, 8 passent ainsi d'une forme sensiblement rectiligne à une forme incurvée en direction de l'embase comme cela apparaît sur les figures 7 et 8. La flexion des deux bras 7 et 8 provoque un effort de traction sur respectivement les deuxièmes extrémités 7B et 8B. Par un effort de réaction des moyens de pivot 12, 13, cet effort de traction se traduit par une rotation des deuxièmes extrémités 7B et 8B autour des moyens de pivot 12, 13, comme indiqué par les flèches F4. Les deux parties 5 et 6 de l'enveloppe étant reliées aux deuxièmes extrémités 7B et 8B, elles pivotent également conformément aux flèches F5. Les nervures 15, 20 permettent de rigidifier la liaison entre les deuxièmes extrémités 7B et 8B et les deux parties 5 et 6 de l'enveloppe, ce qui permet de bien transférer l'effort exercé sur les bras 7 et 8 aux deux parties 5 et 6 de l'enveloppe. Ainsi, les deux parties 5 et 6 de l'enveloppe s'écartent l'une de l'autre et le dentiste peut accéder à la prothèse dentaire. Les deux bras 7, 8 sont flexibles dans la mesure où ils peuvent être suffisamment fléchis par un effort de pincement entre deux doigts de manière à induire un pivotement des deux parties de l'enveloppe.

Comme l'ouverture du dispositif de conditionnement ne requiert que l'usage d'une seule main, le dentiste peut facilement saisir la prothèse dentaire P avec l'autre main sans risque de la faire tomber. Le cas échéant, il peut exercer un effort de torsion sur la prothèse dentaire P de manière à la détacher de la partie sécable S qui reste alors en place dans le support 3.

En configuration ouverte, Les deux bras 7, 8 demeurent maintenus par le moyen de retenue. Les deux parties 5 et 6 de l'enveloppe peuvent s'étendre selon deux axes formant un angle d'environ 180° comme cela apparait sur la figure 8. En variante, la configuration ouverte du dispositif de conditionnement pourrait ne pas correspondre exactement à la configuration illustrée sur la figure 8. Au contraire, en configuration ouverte le dispositif de conditionnement peut se trouver dans une configuration intermédiaire entre la configuration fermée (illustrée sur les figures 2, 4 et 6) et la configuration de la figure 8. Par exemple, la configuration ouverte pourrait également correspondre à la configuration de la figure 7. En configuration ouverte, les deux parties 5 et 6 de l'enveloppe peuvent s'étendre selon deux axes formant, de préférence, un angle supérieur ou égal à 60°, notamment supérieur ou égal à 90°. Dans tous les cas, en configuration ouverte, les deux parties 5 et 6 de l'enveloppe sont suffisamment écartées l'une de l'autre pour permettre un accès aisé à la prothèse dentaire.

Selon une variante de réalisation non représentée, le dispositif de conditionnement pourrait comprendre un seul des deux bras 7, 8 tels que décrit précédemment. Une seule des deux parties 5, 6 de l'enveloppe 4 pourrait alors être fixe relativement à l'embase 2. Le déplacement de la partie 5, 6 reliée à l'unique bras suffirait pour donner un accès à la prothèse dentaire. Dans une telle variante de réalisation les deux parties 5 et 6 de l'enveloppe pourraient avantageusement être dissymétriques. Une telle variante du dispositif de conditionnement pourrait donc être encore plus simple à fabriquer. Toutefois, un dispositif de conditionnement dont les deux parties de l'enveloppe sont mobiles relativement à l'embase, et comprenant deux bras chacun relié à une partie de l'enveloppe, présente l'avantage d'offrir un écartement plus important des deux parties de l'enveloppe lorsqu'elle est en configuration ouverte, ce qui facilite l'accès à la prothèse dentaire.

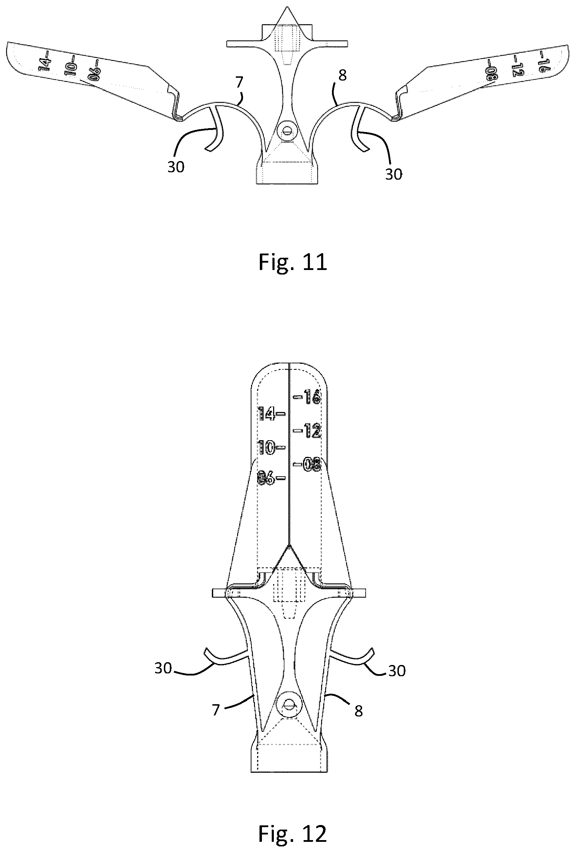

Selon une autre variante de réalisation représentée sur les figures 11 et 12, chaque bras 7, 8 pourrait comprendre une surface d'appui 30 s'étendant sensiblement radialement vers l'extérieur, c'est-à-dire perpendiculairement à l'axe Z. Pour ouvrir le dispositif de conditionnement, l'utilisateur pourrait positionner son pouce sur une face inférieure de l'embase 2 et son index et son majeur respectivement sur les deux surfaces d'appui. Les surfaces d'appui pourraient éventuellement avoir une forme recourbée adaptée pour recevoir l'index et le majeur. Cette prise en main s'apparente à la prise en main d'une seringue. Pour ouvrir le dispositif de conditionnement, l'utilisateur pourrait exercer un effort de traction vers le bas sur chaque surface d'appui (de la même manière qu'on presse une seringue). Cet effort de traction induirait le pivotement des deux parties de l'enveloppe par réaction des deuxièmes extrémités sur les moyens de pivot, comme décrit précédemment.

Les différentes variantes de réalisation décrites peuvent être combinées entre elles. Notamment, la variante de réalisation des figures 11 et 12 peut être réalisée avec un seul bras parmi les deux bras 7 et 8. La variante de réalisation des figures 11 et 12 ainsi que la variante de réalisation avec un seul bras parmi les deux bras 7 et 8 peuvent comprendre un clip de fixation 27 agencé entre les deux parties de l'enveloppe.

## Revendications

1. Dispositif de conditionnement (1) pour une prothèse dentaire (P), comprenant :
- une embase (2) comprenant un support (3) destiné à maintenir la prothèse dentaire,
- une enveloppe (4) formée d'une première partie (5) et d'une deuxième partie (6), et
- au moins un bras (7) comprenant une première extrémité (7A) et une deuxième extrémité (7B), la première extrémité étant reliée à l'embase, la deuxième extrémité étant reliée à la première partie de l'enveloppe,
l'embase (2) comprenant une forme globalement allongée, la première extrémité (7A) de l'au moins un bras étant reliée à une première extrémité (2A) de l'embase, le support (3) étant agencé à une deuxième extrémité (2B) de l'embase, la deuxième extrémité de l'embase étant opposée à la première extrémité de l'embase,
le dispositif de conditionnement étant apte à passer d'une configuration fermée à une configuration ouverte, la première partie (5) et la deuxième partie (6) de
l'enveloppe étant rapprochée l'une de l'autre de manière à envelopper la prothèse dentaire lorsque le dispositif de conditionnement est en configuration fermée, la première partie (5) et la deuxième partie (6) de l'enveloppe étant écartées
l'une de l'autre de manière à accéder à la prothèse dentaire lorsque le dispositif de conditionnement est en configuration ouverte,
l'embase (2) comprenant au moins un moyen de pivot (12) pour l'au moins un bras,
un effort appliqué sur l'au moins un bras entre sa première extrémité (7A) et l'au
moins un moyen de pivot (12) faisant fléchir l'au moins un bras (7) en direction de
l'embase (2), la flexion de l'au moins un bras entraînant le passage du dispositif de conditionnement de sa configuration fermée à sa configuration ouverte.

2. Dispositif de conditionnement (1) selon la revendication précédente, **caractérisé en ce qu'**il comprend un premier bras (7) et un deuxième bras (8), chaque bras comprenant une première extrémité (7A, 7B) et une deuxième extrémité (7B, 8B), la première extrémité de chaque bras étant reliée à l'embase (2), la deuxième extrémité (7B) du premier bras étant reliée à la première partie (5) de l'enveloppe, la deuxième extrémité (8B) du deuxième bras étant reliée à la deuxième partie (6) de l'enveloppe, l'embase comprenant un premier moyen de pivot (12) pour le premier bras et un deuxième moyen de pivot (13) pour le deuxième bras, un effort appliqué sur le premier bras entre sa première extrémité et le premier moyen de pivot et un effort appliqué sur le deuxième bras entre sa première extrémité et le deuxième moyen de pivot entraînant le passage du dispositif de conditionnement de sa configuration fermée à sa configuration ouverte.

3. Dispositif de conditionnement (1) selon l'une des revendications précédentes, **caractérisé en ce qu'**il est une pièce monobloc obtenue par injection plastique.

4. Dispositif de conditionnement (1) selon l'une des revendications précédentes, **caractérisé en ce que** l'embase (2) comprend au moins un moyen de retenue coopérant avec l'au moins un bras pour maintenir le dispositif de conditionnement en configuration fermée.

5. Dispositif de conditionnement (1) selon la revendication précédente, **caractérisé en ce que** le moyen de retenue comprend au moins une patte (16, 17) déformable, la patte étant destinée à se déformer pour permettre le déplacement de l'au moins un bras lorsque le dispositif de conditionnement passe d'une configuration originelle à l'issue de sa fabrication à sa configuration fermée.

6. Dispositif de conditionnement (1) selon l'une des revendications précédentes, **caractérisé en ce qu'**il comprend une mémoire de forme tendant à le faire passer vers sa configuration fermée.

7. Dispositif de conditionnement (1) selon l'une des revendications précédentes,
**caractérisé en ce que** :
- l'embase (2) comprend en outre au moins un logement (21, 22) pour une vis de fixation apte à coopérer avec la prothèse dentaire, et/ou
- la première partie (5) et la deuxième partie (6) de l'enveloppe sont deux demi-coques de l'enveloppe, et/ou
- la première partie (5) et/ou la deuxième partie (6) de l'enveloppe sont fabriquées en matériau plastique transparent et comportent des graduations (23) en relief configurées pour estimer une taille de la prothèse dentaire.

8. Dispositif de conditionnement (1) selon l'une des revendications précédentes,
**caractérisé en ce qu'**il comprend un clip de fixation (27) agencé entre les deux parties (5, 6) de l'enveloppe (4) pour maintenir le dispositif de conditionnement en configuration fermée.

9. Kit comprenant un dispositif de conditionnement (1) selon l'une des
revendications précédentes et une prothèse dentaire (P) fixée au support (3) de l'embase (2).

10. Procédé de fabrication d'un dispositif de conditionnement (1) selon l'une des revendications 1 à 8,
**caractérisé en ce que** le dispositif de conditionnement est fabriqué par injection plastique dans une configuration originelle dans laquelle l'au moins un bras (7) présente une forme en arc de cercle et dans laquelle l'au moins un bras est écarté de l'au moins un moyen de pivot (12) .

11. Procédé de conditionnement d'une prothèse dentaire (P) dans un dispositif de conditionnement (1) selon l'une des revendications 1 à 8, comprenant :
- la fabrication par injection plastique du dispositif de conditionnement, puis
- la fixation d'une prothèse dentaire au support de l'embase, puis
- le rapprochement de la première partie de l'enveloppe et de la deuxième partie de l'enveloppe autour de la prothèse dentaire.

12. Procédé de déconditionnement d'une prothèse dentaire (P) conditionnée dans un dispositif de conditionnement (1) selon l'une des revendications 1 à 8, comprenant une pression contre l'au moins un bras entre sa première extrémité et l'au moins un moyen de pivot (12) pour faire fléchir l'au moins un bras en direction de l'embase (2), la flexion de l'au moins un bras entraînant l'ouverture du dispositif de conditionnement.

## Patentansprüche

1. Verpackungsvorrichtung (1) für eine Zahnprothese (P), umfassend:
- eine Basis (2) mit einer Halterung (3), die dazu bestimmt ist, die Zahnprothese zu tragen,
- eine Hülle (4), gebildet aus einem ersten Teil (5) und einem zweiten Teil (6), und
- mindestens einen Arm (7), der ein erstes Ende (7A) und ein zweites Ende (7B) aufweist, wobei das erste Ende mit der Basis verbunden ist und das zweite Ende mit dem ersten Teil der Hülle verbunden ist,
wobei die Basis (2) eine im Wesentlichen längliche Form aufweist, das erste Ende (7A) des mindestens einen Arms mit einem ersten Ende (2A) der Basis verbunden ist, die Halterung (3) an einem zweiten Ende (2B) der Basis angeordnet ist, wobei das zweite Ende der Basis dem ersten Ende der Basis gegenüberliegt,
wobei die Verpackungsvorrichtung von einer geschlossenen Konfiguration in eine offene Konfiguration überführbar ist, wobei der erste Teil (5) und der zweite Teil (6) der Hülle einander angenähert sind, um die Zahnprothese zu umschließen, wenn sich die Verpackungsvorrichtung in der geschlossenen Konfiguration befindet, und wobei der erste Teil (5) und der zweite Teil (6) der Hülle voneinander beabstandet sind, um Zugang zur Zahnprothese zu ermöglichen, wenn sich die Verpackungsvorrichtung in der offenen Konfiguration befindet,
wobei die Basis (2) mindestens ein Drehmittel (12) für den mindestens einen Arm umfasst, wobei eine auf den mindestens einen Arm zwischen seinem ersten Ende (7A) und dem mindestens einen Drehmittel (12) ausgeübte Kraft bewirkt, dass sich der mindestens eine Arm in Richtung der Basis (2) biegt, wobei das Biegen des mindestens einen Arms bewirkt, dass die Verpackungsvorrichtung von ihrer geschlossenen Konfiguration in ihre offene Konfiguration übergeht.

2. Verpackungsvorrichtung (1) nach dem vorhergehenden Anspruch, **dadurch gekennzeichnet, dass** sie einen ersten Arm (7) und einen zweiten Arm (8) umfasst, wobei jeder Arm ein erstes Ende (7A, 8A) und ein zweites Ende (7B, 8B) aufweist, das erste Ende jedes Arms mit der Basis (2) verbunden ist, das zweite Ende (7B) des ersten Arms mit dem ersten Teil (5) der Hülle verbunden ist und das zweite Ende (8B) des zweiten Arms mit dem zweiten Teil (6) der Hülle verbunden ist, wobei die Basis ein erstes Drehmittel (12) für den ersten Arm und ein zweites Drehmittel (13) für den zweiten Arm umfasst, wobei eine auf den ersten Arm zwischen seinem ersten Ende und dem ersten Drehmittel ausgeübte Kraft sowie eine auf den zweiten Arm zwischen seinem ersten Ende und dem zweiten Drehmittel ausgeübte Kraft bewirken, dass die Verpackungsvorrichtung von ihrer geschlossenen Konfiguration in ihre offene Konfiguration übergeht.

3. Verpackungsvorrichtung (1) nach einem der vorhergehenden Ansprüche, **dadurch gekennzeichnet, dass** sie einstückig ist und durch Kunststoffspritzgießen hergestellt wird.

4. Verpackungsvorrichtung (1) nach einem der vorhergehenden Ansprüche, **dadurch gekennzeichnet, dass** die Basis (2) mindestens ein Haltemittel umfasst, das mit dem mindestens einen Arm zusammenwirkt, um die Verpackungsvorrichtung in einer geschlossenen Konfiguration zu halten.

5. Verpackungsvorrichtung (1) nach dem vorhergehenden Anspruch, **dadurch gekennzeichnet, dass** das Haltemittel mindestens eine verformbare Lasche (16, 17) umfasst, wobei die Lasche dazu bestimmt ist, sich zu verformen, um die Bewegung des mindestens einen Arms zu ermöglichen, wenn die Verpackungsvorrichtung von einer ursprünglichen Konfiguration am Ende ihrer Herstellung in ihre geschlossene Konfiguration übergeht.

6. Verpackungsvorrichtung (1) nach einem der vorhergehenden Ansprüche, **dadurch gekennzeichnet, dass** sie ein Formgedächtnis aufweist, das dazu neigt, sie in ihre geschlossene Konfiguration zu überführen.

7. Verpackungsvorrichtung (1) nach einem der vorhergehenden Ansprüche, **dadurch gekennzeichnet, dass**:
- die Basis (2) ferner mindestens eine Aufnahme (21, 22) für eine Befestigungsschraube umfasst, die mit der Zahnprothese zusammenwirken kann, und/oder
- der erste Teil (5) und der zweite Teil (6) der Hülle zwei Halbschalen der Hülle sind, und/oder
- der erste Teil (5) und/oder der zweite Teil (6) der Hülle aus transparentem Kunststoffmaterial bestehen und erhabene Graduierungen (23) aufweisen, die zur Abschätzung einer Größe der Zahnprothese ausgelegt sind.

8. Verpackungsvorrichtung (1) nach einem der vorhergehenden Ansprüche, **dadurch gekennzeichnet, dass** sie einen Befestigungsclip (27) umfasst, der zwischen den beiden Teilen (5, 6) der Hülle (4) angeordnet ist, um die Verpackungsvorrichtung in einer geschlossenen Konfiguration zu halten.

9. Kit, umfassend eine Verpackungsvorrichtung (1) nach einem der vorhergehenden Ansprüche und eine Zahnprothese (P), die an der Halterung (3) der Basis (2) befestigt ist.

10. Verfahren zur Herstellung einer Verpackungsvorrichtung (1) nach einem der Ansprüche 1 bis 8, **dadurch gekennzeichnet, dass** die Verpackungsvorrichtung durch Kunststoffspritzgießen in einer ursprünglichen Konfiguration hergestellt wird, in der der mindestens eine Arm (7) die Form eines Kreisbogenabschnitts aufweist und in der der mindestens eine Arm von dem mindestens einen Drehmittel (12) beabstandet ist.

11. Verfahren zum Verpacken einer Zahnprothese (P) in einer Verpackungsvorrichtung (1) nach einem der Ansprüche 1 bis 8, umfassend:
- Herstellen der Verpackungsvorrichtung durch Kunststoffspritzgießen, dann
- Befestigen einer Zahnprothese an der Halterung der Basis, dann
- Zusammenführen des ersten Teils der Hülle und des zweiten Teils der Hülle um die Zahnprothese.

12. Verfahren zum Entpacken einer in einer Verpackungsvorrichtung (1) nach einem der Ansprüche 1 bis 8 verpackten Zahnprothese (P), umfassend das Drücken auf den mindestens einen Arm zwischen seinem ersten Ende und dem mindestens einen Drehmittel (12), um zu bewirken, dass sich der mindestens eine Arm in Richtung der Basis (2) biegt, wobei das Biegen des mindestens einen Arms bewirkt, dass sich die Verpackungsvorrichtung öffnet.

## Claims

1. A packaging device (1) for a dental prosthesis (P), comprising:
- a base (2) comprising a support (3) intended to hold the dental prosthesis,
- an envelope (4) formed of a first part (5) and of a second part (6), and
- at least one arm (7) comprising a first end (7A) and a second end (7B), the first end being connected to the base, the second end being connected to the first part of the envelope, the base (2) comprises a generally elongate shape, the first end (7A) of the at least one arm being connected to a first end (2A) of the base, the support (3) being arranged at a second end (2B) of the base, the second end of the base being opposite the first end of the base,
the packaging device being able to transition from a closed configuration to an open configuration, the first part (5) and the second part (6) of the envelope being brought close together to enclose the dental prosthesis when the packaging device is in the closed configuration, the first part (5) and the second part (6) of the envelope being spaced apart from each other to provide access to the dental prosthesis when the packaging device is in the open configuration,
the base (2) comprising at least one pivot means (12) for the at least one arm, a force applied to the at least one arm, between its first end (7A) and the at least one pivot means (12) causing the at least one arm to bend toward the base (2), the bending of the at least one arm causing the packaging device to transition from its closed configuration to its open configuration.

2. The packaging device (1) as claimed in the preceding claim, **characterized in that** it comprises a first arm (7) and a second arm (8), each arm comprising a first end (7A, 8A) and a second end (7B, 8B), the first end of each arm being connected to the base (2), the second end (7B) of the first arm being connected to the first part (5) of the envelope, the second end (8B) of the second arm being connected to the second part (6) of the envelope, the base comprising a first pivot means (12) for the first arm and a second pivot means (13) for the second arm, a force applied to the first arm, between its first end and the first pivot means, and a force applied to the second arm, between its first end and the second pivot means, causing the packaging device to transition from its closed configuration to its open configuration.

3. The packaging device (1) as claimed in either of the preceding claims, **characterized in that** it is in one piece and obtained by plastic injection.

4. The packaging device (1) as claimed in any one of the preceding claims, **characterized in that** the base (2) comprises at least one retention means cooperating with the at least one arm in order to hold the packaging device in a closed configuration.

5. The packaging device (1) as claimed in the preceding claim, **characterized in that** the retention means comprises at least one deformable tab (16, 17), the tab being intended to deform in order to allow the movement of the at least one arm when the packaging device transitions from an original configuration at the end of its manufacture to its closed configuration.

6. The packaging device (1) as claimed in any one of the preceding claims, **characterized in that** it comprises a shape memory that tends to cause it to transition to its closed configuration.

7. The packaging device (1) as claimed in any one of the preceding claims, **characterized in that**:
- the base (2) further comprises at least one housing (21, 22) for a fastening screw able to cooperate with the dental prosthesis, and/or
- the first part (5) and the second part (6) of the envelope are two half-shells of the envelope, and/or
- the first part (5) and/or the second part (6) of the envelope are made of transparent plastic material and have graduations (23) in relief, which are configured for estimating a size of the dental prosthesis.

8. The packaging device (1) as claimed in any one of the preceding claims, **characterized in that** it comprises a fastening clip (27) arranged between the two parts (5, 6) of the envelope (4), for maintaining the packaging device in a closed configuration.

9. A kit comprising a packaging device (1) as claimed in any one of the preceding claims and a dental prosthesis (P) fastened to the support (3) of the base (2).

10. A method for manufacturing a packaging device (1) as claimed in any one of claims 1 to 8, **characterized in that** the packaging device is manufactured by plastic injection in an original configuration in which the at least one arm (7) has the shape of an arc of a circle and in which the at least one arm is spaced apart from the at least one pivot means (12).

11. A method for packaging a dental prosthesis (P) in a packaging device (1) as claimed in any one of claims 1 to 8, comprising:
- manufacturing the packaging device by plastic injection, then
- fastening a dental prosthesis to the support of the base, then
- bringing the first part of the envelope and the second part of the envelope together around the dental prosthesis.

12. A method for unpackaging a dental prosthesis (P) packaged in a packaging device (1) as claimed in any one of claims 1 to 8, comprising pressing against the at least one arm, between its first end and the at least one pivot means (12), in order to cause the at least one arm to bend in the direction of the base (2), the bending of the at least one arm causing the packaging device to open.
